# EUROPEAN PATENT APPLICATION

(11) **EP 4 338 794 A1**
(43) Date of publication of application: **20.03.2024**
(21) Application number: 23196967.6
(22) Date of filing: 12.09.2023
(51) Int. Cl.: A61N 5/06

(54) **TRANSCRANIAL DEVICE**

(30) Priority: 15.09.2022 AU 2022902675; 08.09.2023 AU 2023226789
(71) Applicant: Symbyx Pty Ltd, Neutral Bay, NSW 2089 (AU)
(72) Inventor: MARKMAN, Wayne, Neutral Bay, 2089 (AU)
(74) Representative: Sach, Greg Robert

(57) **Abstract**

A transcranial device for irradiating a cranium of a user includes a power source, and a headset configured to receive power from the power source. The headset includes a positioning structure including one or more irradiation sources and a control module located on a substrate structure. The control module is configured to modify operational characteristics of the one or more irradiation sources. The substrate structure includes a central segment, and one or more ribs extending from the central segment. The one or more ribs include one or more respective expanded portions at one or more respective distal ends of the one or more respective ribs. The one or more ribs are pliable, such that, the one or more ribs are configured to be bent for locating and fastening the headset onto a head of a user, using one or more first fastening structures, and a second fastening structure.

## Description

### CROSS-REFERENCE TO RELATED APPLICATIONS

This patent application claims the benefit of priority from Australian Patent Application numbered 2023226789 filed on 08 September 2023 which takes priority from the Australia Provisional Patent application 2022902675 filed on September 15, 2022, incorporated by reference herein.

### TECHNICAL FIELD

The present invention generally relates to electromagnetic radiation-based medical devices. More particularly, the present invention relates to a transcranial device used for treating neurological diseases.

### BACKGROUND ART

It is to be understood that, if any prior art is referred to herein, such reference does not constitute an admission that the prior art forms a part of the common general knowledge in the art, in Australia or any other country.

Photo-biomodulation therapy (PBM; which is also known as light therapy, low-intensity laser therapy, or low-level laser therapy) has been used in humans for a wide range of conditions, such as musculoskeletal disorders, inflammatory conditions, and neurogenesis. PBM is a safe, non-invasive, and non-thermal modality that is based on a substantial body of research dating back to the 1960s. The mechanisms of PBM may involve the stimulation of mitochondria by the absorption of photons in cytochrome c oxidase, resulting in increased ATP production, leading to reduced oxidative stress, anti-inflammatory effects, improved cellular energy, increased synthesis of enzymes, restoration of damaged and creation of new synapses, stimulation of new neuronal and vascular growth, and increased focal cerebral blood flow due to vascular and lymphatic vasodilation.

It has been found through several studies that irradiation of posterior portions of the brain helps in the repair and enhancement of cognitive function and repair of damaged brain cells in patients suffering from Parkinson's Disease (PD). Recent research has reported neuroprotection against brain cell degeneration, stimulated by PBM in animal models with PD. Further potential applications of the irradiation of the frontal portions of the brain include treatment of Attention Deficit/Hyperactivity Disorder (ADHD), Post Traumatic Stress Disorder (PTSD), Traumatic Brain Injury (TBI), depression, and concussive injuries. Transcranial irradiation has also been known to treat other psychiatric disorders such as Major Depressive Disorder (MDD) and improve stroke outcomes.

There are existing devices that also utilise the mechanisms of PBM therapy. Such devices are typically known to have 40 Red LEDs and 40 Infrared (IR) LEDs being driven at peak currents of 400 mA at 40 HZ, with a duty cycle of 32%. However, several other devices, in contrast, offer a combination of one or more transcranial Photo-biomodulation (tPBM) devices and a proprietary nasal applicator.

### SUMMARY

According to a first aspect of the present invention, there is provided a transcranial device for irradiating a cranium of a user. The transcranial device includes a power source, and a headset configured to receive power from the power source. The headset includes a positioning structure including one or more irradiation sources and a control module located on a substrate structure. The control module is configured to modify operational characteristics of the one or more irradiation sources. The substrate structure includes a central segment and one or more ribs extending longitudinally and outwardly from the central segment. The one or more ribs include one or more respective proximal ends attached to the central segment and one or more respective distal ends located longitudinally at one or more respective predetermined distances from the central segment. The one or more ribs include one or more respective expanded portions at the one or more respective distal ends of the one or more respective ribs. The one or more ribs are pliable, such that, the one or more ribs are configured to be bent for locating and fastening the headset onto a head of a user, using one or more first fastening structures and a second fastening structure.

In one embodiment of the invention, the power source includes one or more rechargeable batteries.

In one embodiment of the invention, the power source includes a power adaptor including an Analog to Digital (AC/DC) converter. The power adaptor is configured to receive power from an Alternating Current (AC) based power supply.

In one embodiment of the invention, the power source is attached to the positioning structure.

In one embodiment of the invention, the one or more irradiation sources are selected from a group consisting of Light Emitting Diodes (LEDs), lasers, and incandescent sources of electromagnetic radiation.

In one embodiment of the invention, the plurality of irradiation sources is configured to emit electromagnetic radiation of red-light wavelengths (630 nm - 670 nm) and Infrared radiation wavelengths (800 nm - 820 nm).

In one embodiment of the invention, the one or more irradiation sources include twenty (20) LEDs configured to emit red light of wavelengths ranging between 630 nm and 670 nm, and twenty (20) LEDs configured to emit infrared radiation of wavelengths ranging between 800 nm and 820 nm.

In one embodiment of the invention, the control module is located in the central segment.

In one embodiment of the invention, the control module is covered with a protective pad made from a non-conducting material.

In one embodiment of the invention, the non-conducting material is selected from a group consisting of Acrylonitrile Butadiene Styrene (ABS), Polyvinyl Chloride (PVC), High-Density Polyethylene (HDPE), Low-Density Polyethylene (LDPE), and combinations thereof.

In one embodiment of the invention, the control module includes a communication interface configured to wirelessly connect to an external programmable device.

In one embodiment of the invention, the control module is configured to operate the one or more irradiation sources in pulsating mode with a pulsating frequency of 40 Hz and a peak drive current of 400 mA.

In one embodiment of the invention, the control module further includes a four (4) - way Dual In-line Package (DIP) switch configured to allow the user to select an operational state from sixteen (16) preconfigured operational states.

In one embodiment of the invention, the substrate structure is made up of fibreglass and coated with an impermeable solder-resistant material.

In one embodiment of the invention, the one or more ribs include a first rib extending rearwardly from the central segment, the first rib configured to extend over a frontal bone and an occipital bone of the cranium. The one or more ribs further include a second rib extending rearwardly and obliquely towards a right side when in use. The second rib is the configured to extend over a right parietal bone, a right temporal bone, a right mastoid process and the occipital bone of the cranium. Further, the one or more ribs include a third rib extending rearwardly and obliquely towards a left side when in use. The third rib is configured to extend over a left parietal bone, a left temporal bone, a left mastoid process and the occipital bone of the cranium. Furthermore, the one or more ribs include a fourth rib extending laterally towards the right side when in use. The fourth rib is configured to extend over the frontal bone and a right sphenoid bone of the cranium. Also, the one or more ribs include a fifth rib extending laterally towards the left side when in use. The fifth rib is configured to extend over the frontal bone and a left sphenoid bone of the cranium.

In one embodiment of the invention, each one of the one or more expanded portions includes one or more apertures, such that, for locating and fastening the headset onto the head of the user, respective apertures of the one or more expanded portions are configured to be overlapped and fastened using the one or more first fastening structures.

In one embodiment of the invention, the one or more first fastening structures include screws and/or bolts.

In one embodiment of the invention, the central segment includes a notch, and the second fastening structure includes a T-strap with ends provided with loop and hook fasteners. The T-strap includes a head portion, and a body portion. The head portion is configured to connect with the notch to loop around the body portion, to fasten the body portion upwardly across a periphery of a forehead of the user. Also, a transverse fastening strap is configured to fasten the body portion laterally around the periphery of the forehead.

In one embodiment of the invention, the transcranial device further includes a nasal driver for nasal application, the nasal driver configured to operate the one or more irradiation sources with a pulsating frequency of 10 Hz.

According to a second aspect of the present invention, there is provided a transcranial device for irradiating a cranium of a user. The transcranial device includes a power source, and a headset configured to receive power from the power source. The headset includes a positioning structure including one or more irradiation sources and a control module located on a substrate structure. The control module is configured to modify operational characteristics of the one or more irradiation sources. The substrate structure includes a central segment, and one or more ribs extending longitudinally and outwardly from the central segment, the one or more ribs include one or more respective proximal ends attached to the central segment and one or more respective distal ends located longitudinally at one or more respective predetermined distances from the central segment. The one or more ribs are pliable, such that, the one or more ribs are configured to be bent for locating and fastening the headset onto a head of a user, using one or more first fastening structures and a second fastening structure. Also, the one or more ribs include a first rib extending rearwardly from the central segment, the first rib configured to extend over a frontal bone and an occipital bone of the cranium. Furthermore, the one or more ribs include a second rib extending rearwardly and obliquely towards a right side when in use, the second rib configured to extend over a right parietal bone, a right temporal bone, a right mastoid process and the occipital bone of the cranium. The one or more ribs further include a third rib extending rearwardly and obliquely towards a left side when in use, the third rib configured to extend over a left parietal bone, a left temporal bone, a left mastoid process and the occipital bone of the cranium. Furthermore, the one or more ribs include a fourth rib extending laterally towards the right side when in use, the fourth rib configured to extend over the frontal bone and a right sphenoid bone of the cranium. Also, the one or more ribs include a fifth rib extending laterally towards the left side when in use, the fifth rib configured to extend over the frontal bone and a left sphenoid bone of the cranium.

In the context of the specification, the term "processor" refers to one or more of a microprocessor, a microcontroller, a general-purpose processor, a System on a Chip (SoC), a Digital Signal Processor (DSP), a Tensor Processing Unit (TPU), a Field Programmable Gate Array (FPGA) or an Application Specific Integrated Circuit (ASIC), and the like.

In the context of the specification, the phrase "memory unit" refers to volatile storage memory, such as Static Random Access Memory (SRAM) and Dynamic Random Access Memory (DRAM) of types such as Asynchronous DRAM, Synchronous DRAM, Double Data Rate SDRAM, Rambus DRAM, and Cache DRAM, etc.

In the context of the specification, the phrase "storage device" refers to a non-volatile storage memory such as EPROM, EEPROM, flash memory, or the like.

In the context of the specification, the phrase "communication interface" refers to a device or a module enabling direct connectivity via wires and connectors such as USB, HDMI, VGA, or wireless connectivity such as Bluetooth or Wi-Fi, or Local Area Network (LAN) or Wide Area Network (WAN) implemented through TCP/IP, IEEE 802.x, GSM, CDMA, LTE, or other equivalent protocols.

In the context of this specification, terms like "light", "radiation", "irradiation", "emission" and "illumination", etc. refer to electromagnetic radiation in frequency ranges varying from the Ultraviolet (UV) frequencies to Infrared (IR) frequencies and wavelengths, wherein the range is inclusive of UV and IR frequencies and wavelengths. It is to be noted here that UV radiation can be categorized in several manners depending on respective wavelength ranges, all of which are envisaged to be under the scope of this invention. For example, UV radiation can be categorized as, Hydrogen Lyman-? (122-121 nm), Far UV (200-122 nm), Middle UV (300-200 nm), Near UV (400-300 nm). The UV radiation may also be categorized as UVA (400-315 nm), UVB (315-280 nm), and UVC (280-100 nm) Similarly, IR radiation may also be categorized into several categories according to respective wavelength ranges which are again envisaged to be within the scope of this invention. A commonly used subdivision scheme for IR radiation includes Near IR (0.75-1.4 µm), Short-Wavelength IR (1.4-3 µm), Mid-Wavelength IR (3-8 µm), Long-Wavelength IR (8-15 µm) and Far IR (15-1000 µm).

In the context of the specification, a "polymer" is a material made up of long chains of organic molecules (having eight or more organic molecules) including, but not limited to, carbon, nitrogen, oxygen, and hydrogen as their constituent elements. The term polymer is envisaged to include both naturally occurring polymers such as wool, and synthetic polymers such as polyethylene and nylon.

In the context of the specification, "Light Emitting Diodes (LEDs)" refer to semiconductor diodes capable of emitting electromagnetic radiation when supplied with an electric current. The LED are characterized by their superior power efficiencies, smaller sizes, rapidity in switching, physical robustness, and longevity when compared with incandescent or fluorescent lamps. In that regard, the plurality of LEDs may be through-hole type LEDs (generally used to produce electromagnetic radiations of red, green, yellow, blue and white colours), Surface Mount Technology (SMT) LEDs, Bi-colour LEDs, Pulse Width Modulated RGB (Red-Green-Blue) LEDs, and high-power LEDs, etc.

Materials used in the one or more LEDs may vary from one embodiment to another depending upon the frequency of radiation required. Different frequencies can be obtained from LEDs made from pure or doped semiconductor materials. Commonly used semiconductor materials include nitrides of Silicon, Gallium, Aluminium, and Boron, and Zinc Selenide, etc. in pure form or doped with elements such as Aluminium and Indium, etc. For example, red and amber colours are produced from Aluminium Indium Gallium Phosphide (AlGaInP) based compositions, while blue, green, and cyan use Indium Gallium Nitride based compositions. White light may be produced by mixing red, green, and blue lights in equal proportions, while varying proportions may be used for generating a wider colour gamut. White and other coloured lightings may also be produced using phosphor coatings such as Yttrium Aluminium Garnet (YAG) in combination with a blue LED to generate white light and Magnesium doped potassium fluorosilicate in combination with blue LED to generate red light. Additionally, near Ultraviolet (UV) LEDs may be combined with europium-based phosphors to generate red and blue lights and copper and zinc doped zinc sulphide-based phosphor to generate green light.

In addition to conventional mineral-based LEDs, one or more LEDs may also be provided on an Organic LED (OLED) based flexible panel or an inorganic LED-based flexible panel. Such OLED panels may be generated by depositing organic semiconducting materials over Thin Film Transistor (TFT) based substrates. Further, discussion on generation of OLED panels can be found in Bardsley, J. N (2004), "International OLED Technology Roadmap", IEEE Journal of Selected Topics in Quantum Electronics, Vol. 10, No. 1, that is included herein in its entirety, by reference. An exemplary description of flexible inorganic light-emitting diode strips can be found in granted U.S. Pat. No. 7,476,557 B2, titled "Roll-to-roll fabricated light sheet and encapsulated semiconductor circuit devices", which is included herein in its entirety, by reference.

In several embodiments, the one or more LEDs may also be micro-LEDs described through U.S. Pat. Nos. 8,809,126 B2, 8,846,457 B2, 8,852,467 B2, 8,415,879 B2, 8,877,101 B2, 9,018,833 B2 and their respective family members, assigned to NthDegree Technologies Worldwide Inc., which are included herein by reference, in their entirety. The one or more LEDs, in that regard, may be provided as a printable composition of the micro-LEDs, printed on a substrate.

### BRIEF DESCRIPTION

Embodiments will now be described by way of example only, with reference to the accompanying drawings in which:
FIG. 1A illustrates an anatomy of a cranium of a human being;
FIG. 1B illustrates a top perspective view of a transcranial device, a headset of the transcranial device being in a disengaged condition, in accordance with an embodiment of the present invention;
FIG. 2 illustrates a rear perspective view of the transcranial device of FIG. 1B, located on a head of a user, in accordance with an embodiment of the present invention;
FIG. 3 illustrates a rear perspective view of a headset being in an engaged condition, in accordance with another embodiment of the present invention;
FIG. 4 illustrates a right-side perspective view of the headset of FIG. 3;
FIG. 5 illustrates a left side perspective view of the headset of FIG. 3;
FIG. 6 illustrates a front perspective view of the headset of FIG. 3, in use;
FIG. 7 illustrates a right-side perspective view of the headset of FIG. 3, in use;
FIG. 8 illustrates a front perspective view of a fastening structure of the headset of FIG. 3, in use;
FIG. 9 illustrates a perspective view of several components of the transcranial device, in accordance with an embodiment of the present invention; and
FIG. 10 illustrates a logical block diagram of an electronic circuitry of the transcranial device, in accordance with an embodiment of the invention.

Throughout the figures in the drawings, similar reference letters and numerals are utilized to denote similar corresponding parts.

### DETAILED DESCRIPTION

In the following detailed description, reference is made to accompanying drawings which form a part of the detailed description. The illustrative embodiments described in the detailed description, depicted in the drawings and defined in the claims, are not intended to be limiting. Other embodiments may be utilised and other changes may be made without departing from the spirit or scope of the subject matter presented. It will be readily understood that the aspects of the present invention, as generally described herein and illustrated in the drawings may be arranged, substituted, combined, separated and designed in a wide variety of different configurations, all of which are contemplated in this invention.

Embodiments of the present invention provide a transcranial device for treating patients with neurological diseases such as Parkinson's disease. In that regard, the transcranial device is envisaged to include a headset and a power source connected to the headset using a power cable. However, in several embodiments of the invention, the power source may be provided integrally with the headset. The headset has been provided with a positioning structure including a substrate structure made from a strong but pliable material. For example, the substrate structure may be made up of fibreglass. The substrate structure may include a central segment where the electronic circuitry of the headset may be located. Further, the substrate structure may include several ribs extending longitudinally outwards from the central segment. The ribs are envisaged to include proximal ends attached to the central segment and distal ends located at predetermined distances from the central segment. The ribs may include respective expanded portions at the distal ends of the respective ribs. The ribs are envisaged to be pliable so that the headset can conform to different shapes and sizes of heads of several users. Moreover, the respective expanded portions may further include apertures for facilitating fastening between the ribs using fastening structures while the headset is being affixed on the head of the user. The fastening structures may include screws, bolts, belts and straps including loop and hook fasteners, buckles, snap-fit arrangements, and the like. Another fastening structure, such as a T-strap structure, may be provided to facilitate the fastening of the headset to the head of the user.

Several irradiation sources such as Light Emitting Diodes (LEDs), lasers, and other equivalent light sources may be located on the ribs including on the respective expanded portions, such that the irradiation sources align with key points/locations of the brain for stimulating predefined key areas of the brain. The irradiation sources may be selected to emit electromagnetic radiation at several different wavelengths depending upon the intended application of the transcranial device. For example, the headset may include equal numbers of LEDs emitting red and infrared wavelengths. Operational characteristics, such as pulse rate, duty cycle, etc. of the irradiation sources may be controlled by a control module located in the central segment or any other location of the positioning structure. In that regard, the control module may include a processor, memory unit and storage devices with machine-readable instructions, communication interfaces, clocks, sensors, DC/DC converters, etc. Power for the transcranial device may be provided by a power source that may be connected to the headset using a power cable. As discussed above, the power source may also be integrally located in the headset. Further, the power source may be an Alternating Current (AC) based power source with an AC/DC converter, or Direct Current (DC) based batteries such as Lithium-based rechargeable batteries. It will be understood by the person skilled in the art that the transcranial device elucidated in the following discussion may be utilised in the treatment of several medical conditions or diseases or for the treatment of users with no known conditions.

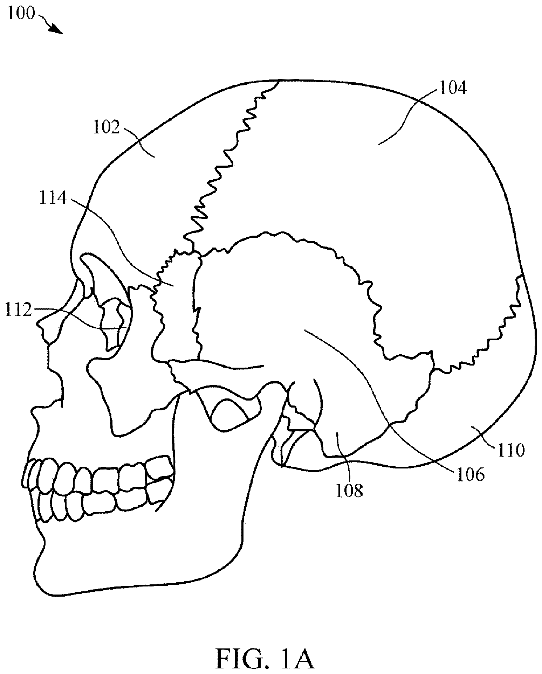

Several embodiments of the present invention will now be elucidated with reference to FIGS 1A-10.

**FIG. 1A** illustrates the anatomy of a cranium **100** of a human being. The cranium **100** includes a total of eight (8) bones including a frontal bone **102,** two parietal bones **104,** two temporal bones **106,** an occipital bone **110**, an ethmoid bone **112,** and a sphenoid bone **114.** The frontal bone **102** makes up the forehead and upper portions of the eye sockets of a human skull. The shape of the frontal bone **102** plays a significant role in the visual appearance of a person. The frontal bone **102** includes openings that allow blood vessels and nerves to reach the eyebrows, eyelids, and frontal sinuses. The two parietal bones **104** are located at two upper lateral sides of the human skull, behind the frontal bone **102.** The two parietal bones **104** are connected to the frontal bone **102,** the sphenoid bone **114,** the two temporal bones **106,** and the occipital bone **110.** The two temporal bones **106** are located on two lower lateral sides of the human skull under a corresponding parietal bone of the two parietal bones **104.** The two temporal bones **106** include two respective mastoid processes **108** portions. The two mastoid processes **108** are conical/pyramidal projections that serve as insertion sites for many muscles in the head and neck region. The two mastoid processes **108** also contain air-filled spaces mastoid air cells.

The occipital bone **110** is located at the back side of the cranium **100** and makes up the posterior and the base portion of the cranium **100.** The occipital bone **110** includes an opening called foramen magnum, that allows the spinal cord to connect with the brain. The ethmoid bone **112** is an irregular bone located in front of the sphenoid bone **114** and makes up a part of the nasal cavity. The ethmoid bone **112** separates the nasal cavity from the brain. The sphenoid bone **114** is an irregular bone located below the frontal bone **102.** The sphenoid bone **114** spans the width of the skull and forms a large part of the base of the skull. The sphenoid bone **114** forms an anterior portion of the cranium **100.** Several embodiments of the invention as discussed in the following discussion have been designed to be located on the cranium **100** of the user. However, a person skilled in the art would appreciate that the embodiments discussed in the following discussion may be adapted to be located on any other portion of the human body, without departing from the scope of the invention.

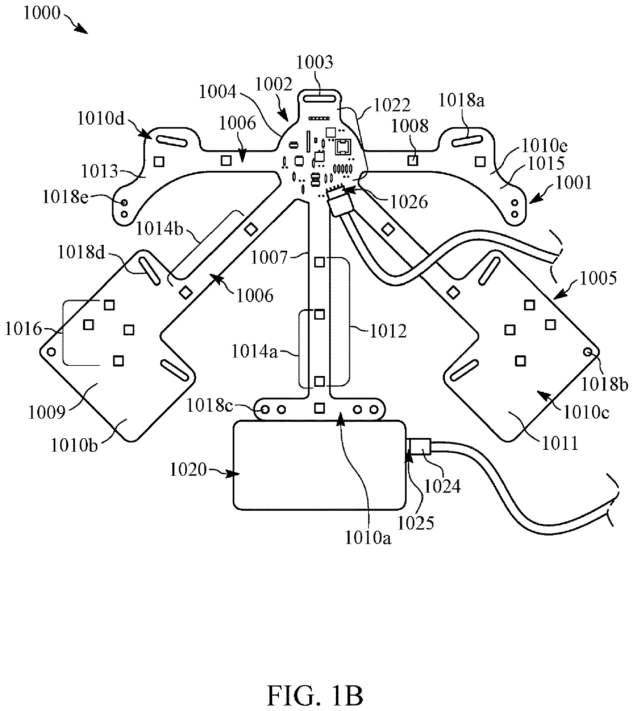

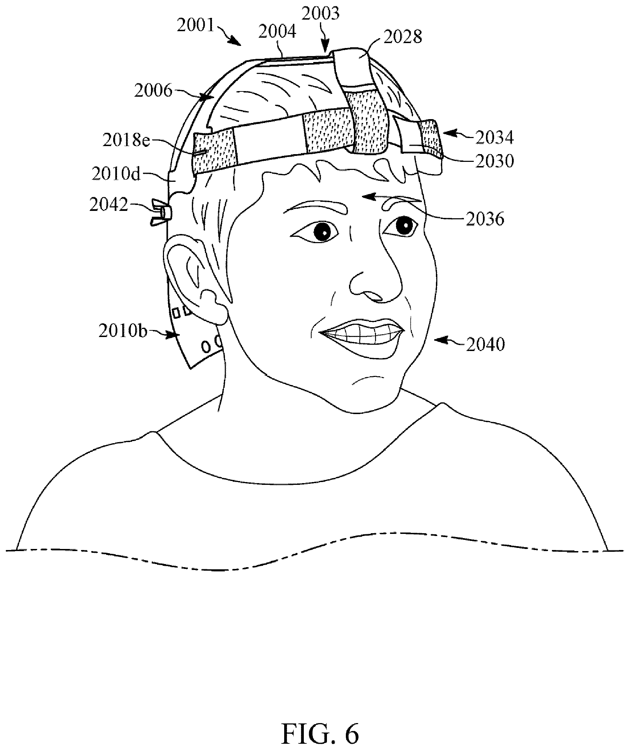

**FIG. 1B** illustrates a top perspective view of a transcranial device **1000** (hereinafter also referred to as "the device 1000"), in accordance with an embodiment of the present invention. The device **1000** includes a headset **1001.** The headset **1001** includes a positioning structure **1002** and a second fastening structure (*See* ***FIGS 6-******8*).** The positioning structure **1002** is configured to locate the headset **1001** over the cranium **100** of a user, and the second fastening structure is configured to fasten the headset **1001** to a head portion of the user.

The headset **1001** has been illustrated to be in a disengaged condition. Further, the transcranial device **1000** includes one or more irradiation sources **1008** arranged on the positioning structure 1002. The one or more irradiation sources **1008** are configured to emit electromagnetic radiations. In that regard, the one or more irradiation sources **1008** may include Light Emitting Diodes (LEDs), lasers, and other equivalent light sources, etc. In the illustrated form, the positioning structure **1002** comprises a substrate structure **1005** including a central segment **1004** and one or more ribs **1006** extending outwardly from the central segment **1004.** In the embodiment illustrated in **FIG. 1****,** the one or more ribs **1006** include five (5) ribs spaced apart and extending outwardly from the central segment **1004.** In the illustrated embodiment, the five (5) ribs of the one or more ribs **1006** include a first rib **1007** extending rearwardly from the central segment **1004.** The first rib **1007** is configured to extend over a frontal bone and the occipital bone of the cranium **100** of the user. Further, the one or more ribs **1006** includes a second rib **1009** extending rearwardly and obliquely towards the right side when in use. The second rib **1009** is configured to extend over a right parietal bone, a right temporal bone, a right mastoid process and the occipital bone of the cranium **100.** A third rib **1011** extending rearwardly and obliquely towards the left side, when in use, is configured to extend over a left parietal bone, a left temporal bone, a left mastoid process and the occipital bone of the cranium **100.** A fourth rib **1013** extending laterally towards the right side, when in use, is configured to extend over the frontal bone and a right sphenoid bone of the cranium **100.** A fifth rib **1015** extending laterally towards the left side, when in use, is configured to extend over the frontal bone and a left sphenoid bone of the cranium **100.** However, a skilled artisan would appreciate that multiple alternative embodiments of ribs fall within the scope of the invention.

The first **1007,** the second **1009,** the third **1011,** the fourth **1013,** and the fifth **1015** ribs include respective first **1010a,** second **1010b,** third **1010c,** fourth **1010d,** and fifth **1010e** expanded portions at respective distal ends of the first **1007,** the second **1009,** the third **1011,** the fourth **1013,** and the fifth **1015** ribs. The ribs shown in the first embodiment of the invention extend from a node end located at the central segment **1004** to an expanded portion located at the distal end of each rib of the one or more ribs **1006.** Several fastening holes and/or slots (for example, for receiving screws, bolts, belts, chains, etc.), snap-fit arrangements, loop and hook fasteners, etc. may be provided in the first **1010a,** the second **1010b,** the third **1010c,** the fourth **1010d,** and the fifth **1010e** expanded portions for allowing the fastening of the headset **1001** to the head of the user.

Furthermore, the one or more irradiation sources **1008** may, at least in part, be located at the first **1010a,** the second **1010b,** the third **1010c,** the fourth **1010d,** and the fifth **1010e** expanded portions. For this reason, the first **1010a,** the second **1010b,** the third **1010c,** the fourth **1010d,** and the fifth **1010e** expanded portions may be of various shapes and configurations to allow the one or more irradiation sources **1008** to better align with the desired points on the head of the user. In several embodiments of the invention, the first **1010a,** the second **1010b,** the third **1010c,** the fourth **1010d,** and the fifth **1010e** expanded portions may be embodied in shapes selected from a group consisting of a notch shape, a paddle shape, and a T shape. However, a skilled artisan would appreciate that other shapes and configurations of the first **1010a,** the second **1010b,** the third **1010c,** the fourth **1010d,** and the fifth **1010e** expanded portions not illustrated herein may also be applicable for intent and purposes of the present invention and may include, for example, a boomerang shape, a hook shape, a triangle or square or rectangle shape etc as best suited to the purpose of spacing apart or grouping the one or more irradiation sources **1008.**

The one or more irradiation sources **1008** may be arranged along the positioning structure **1002** in, for example, clusters **1016** or in a spaced relationship **1012.** When arranged in the spaced relationship **1012,** the one or more irradiation sources **1008** may form evenly spaced rows at distances **1014a** or **1014b,** or at random distances along the one or more ribs **1006.** In several alternate embodiments of the invention, the one or more irradiation sources **1008** do not have to be evenly aligned or spaced as they may be offset or misaligned or placed in locations along the positioning structure **1002** that align with the desired areas on the head of the user, when in use.

The one or more irradiation sources **1008** may be configured to emit electromagnetic radiation with wavelengths lying in the Infrared and/or red colour band of the electromagnetic spectrum. In several embodiments of the invention, the wavelengths of the electromagnetic radiation emitted by the one or more irradiation sources **1008** may vary between 800 nm and 820 nm. In several alternate embodiments of the invention, the wavelengths may vary between 630 nm and 670 nm. In several alternate embodiments, the one or more irradiation sources **1008** may be divided into a first group and a second group, where the first group may be configured to emit the electromagnetic radiation with the wavelengths lying between **800** nm and **820** nm, and the second group may be configured to emit the electromagnetic radiation with the wavelengths lying between **630** nm and **670** nm. The transcranial device **1000** illustrated in **FIG. 1** includes twenty (20) irradiation sources **1008** with the wavelengths lying between 800 nm and 820 nm, and twenty (20) irradiation sources **1008** with the wavelengths lying between 630 nm and 670 nm. However, a skilled artisan would appreciate that alternative arrays of the one or more irradiation sources **1008** may also be applied without departing from the scope of the invention.

The positioning structure **1002** may in some forms consist of one or more apertures **1018a, 1018b, 1018c, 1018d,** and **1018e** located in the substrate structure **1005** to assist in allowing the transcranial device **1000** to be fastened to the head of the user, using fastening means such as snap-fit arrangements, loop and hook fasteners, screws/bolts and nuts, etc. The one or more apertures **1018a, 1018b, 1018c, 1018d,** and **1018e** in the illustrated form, are located in the one or more ribs **1006** and may be in forms selected from a group consisting of slots, slits, holes, and combinations thereof. Other variations of the one or more apertures **1018a, 1018b, 1018c, 1018d,** and **1018e** not illustrated herein may include perforations, notches, grooves, tabs or pinholes. For example, the central segment **1004** includes a notch **1003** as a corresponding aperture provided on the central segment **1004.** It will be understood by a person skilled in the art that alternative types of apertures are available and fall within the scope of the invention.

The transcranial device **1000** further includes a control module **1022** located in the central segment **1004.** The control module 1022 is configured to monitor and control operational characteristics of the one or more irradiation sources **1008.** It will be understood that in alternate embodiments of the invention, the control module **1022** may be located in other areas relative to the transcranial device **1000.** For example, the control module **1022** may be located on any one or more of the first **1010a,** the second **1010b,** the third **1010c,** the fourth **1010d,** and the fifth **1010e** expanded portions or on an external surface of the central segment **1004** (i.e., facing away from the head of the user and towards the ambient), or be provided as a component separate from the positioning structure **1002.** The control module **1022** may advantageously be fully insulated from any contact with the scalp of the user via a round protective pad lined with a suitable non-conducting material, such as polymers including but not limited to Acrylonitrile Butadiene Styrene (ABS), Polyvinyl Chloride (PVC), High-Density Polyethylene (HDPE), Low-Density Polyethylene (LDPE), and the like.

The control module **1022** and the one or more irradiation sources **1008** may be powered through a power source **1020** connected via a cable **1024** which connects an outlet port **1025** of the power source **1022** to an inlet port **1026** of the control module **1022.** In several embodiments of the invention, the power source **1020** may include rechargeable batteries such as Lithium-Ion batteries, Lithium-Polymer batteries, Nickel-metal-hydride batteries, and the like. The power capacities of the rechargeable batteries may vary from one implementation of the present invention to another implementation. For example, a typical total power capacity may vary between 7500 mAh and 10000 mAh. In several alternate embodiments of the invention, the power source **1020** may configured to draw electrical power from an Alternating Current (AC) power supply system.

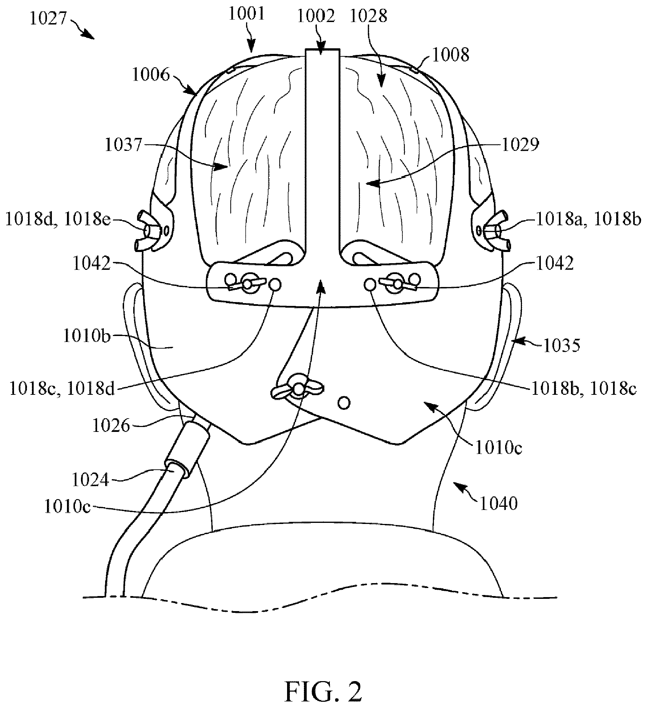

**FIG. 2** illustrates a rear perspective view of an implementation **1027** of the headset **1001** of the transcranial device **1000** of **FIG. 1B****,** located on a head **1037** of a user **1040,** in accordance with an embodiment of the present invention. Here the positioning structure **1002** is bent or otherwise shaped such that the expanded portions **1010b** and **1010c** at least partially overlap and are connected via one or more fastening structures **1042.** The example illustrated herein is the one or more first fastening structures **1042** in the form of plastic screws. In other examples or configurations, not illustrated herein, the one or more first fastening structures **1042** may be in the form of, for example, nuts and bolts, screws, snap-fit arrangement, loop and hook fasteners, or other suitable fastening arrangements and be made from materials with suitable biocompatible and mechanical properties (e.g., polymers, metal).

The positioning structure **1002** is configured to be positioned and to be shaped to fit around the head **1037** of the user **1040.** The substrate structure **1005** of the positioning structure **1002** and the one or more apertures **1018a, 1018b, 1018c, 1018d,** and **1018e** therein are configured such that the one or more ribs **1006** may be affixed together to form a cap, dome, shell or bowl shape configured to form a close fit with the head **1037** of the user **1040.** The positioning structure **1002,** while in use, aligns with at least one of the one or more irradiation sources **1008** with one or more points on the head **1037** following a predefined treatment protocol.

For example, as illustrated in **FIG. 2****,** the expanded portions **1010b** of the second rib **1009** and **1010c** of the third rib **1011** align at least a part of the one or more irradiation sources **1008** (not visible) to the mastoid processes **108** of the head **1037.** Moreover, the central segment **1004,** the first rib **1007,** the fourth rib **1013,** and the fifth rib **1015** align at least a part of the irradiation sources **1008** to areas along the cortical regions of the head **1037.** For example, the cortical regions may include a top or a superior region **1028** and a back or a posterior region **1029.** Furthermore, the one or more apertures **1018a, 1018b, 1018c, 1018d,** and **1018e** may be used to align, receive and/or join the one or more ribs **1006** with several fastening mechanisms **1042** to retain the positioning structure **1002** in shape to be located around a head of the user.

The positioning structure **1002** is configured to support the one or more irradiation sources **1008** such that the transcranial device **1000** aligns at least one of the one or more irradiation sources **1008** with one or more points on the head **1037** to then stimulate the desired areas on the head **1037.** The desired areas may consist of the top or the superior region **1028,** the back or the posterior region **1029** and/or the mastoid process(es) **108** located behind the ears **1035.** In other variations of the invention, the one or more irradiation sources **1008** may be directed to other points across the, for example, ventral and/or cortical regions of the head **1037.**

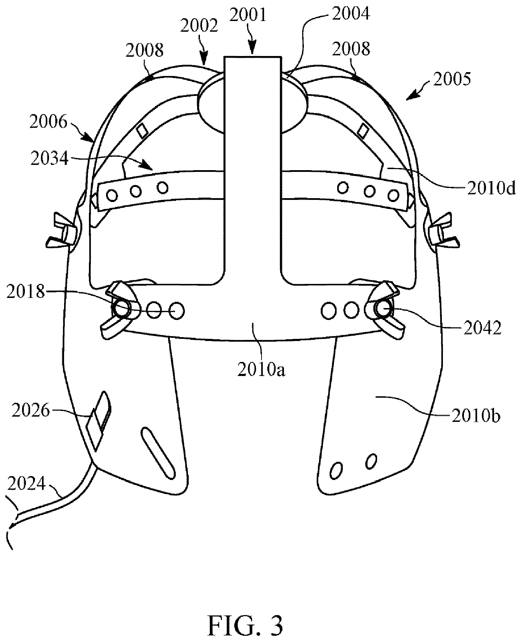

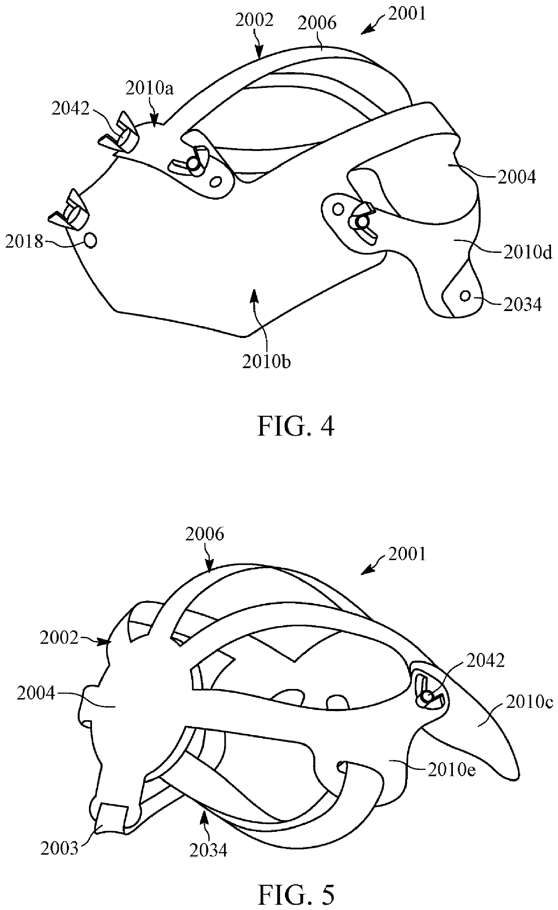

**FIG. 3** illustrates a rear perspective view of a headset **2001** being in an engaged condition, in accordance with another embodiment of the present invention. **FIG. 4** illustrates a right-side perspective view of the headset **2001** of **FIG. 3****.** **FIG. 5** illustrates a left-side perspective view of the headset **2001** of **FIG. 3****.** The headset **2001** includes one or more irradiation sources **2008** arranged on a positioning structure **2002.** In the illustrated form, the positioning structure **2002** comprises a substrate structure **2005** including a central segment **2004** and one or more ribs **2006** extending outwardly from the central segment **2004.** The one or more ribs **2006** include one or more expanded portions **2010a, 2010b, 2010c, 2010d**, and **2010e** in an arrangement similar to the arrangement of the one or more expanded portions **1010a**, **1010b**, **1010c**, **1010d**, and **1010e** illustrated in **FIG. 1A****.**

The positioning structure **2002** includes one or more apertures **2018** provided in the one or more expanded portions **2010a**, **2010b**, **2010c**, **2010d**, and **2010e** and configured to fasten the overlapping ribs of the one or more ribs **2006,** with each other, using one or more first fastening structures **2042.** Also, the central segment **2004** includes a notch **2003.** The headset **2001** may be powered using a cable **2024** connected to a power source (not shown) at one end of the cable **2024,** and an inlet port **2026** of the headset **2001** at another end of the cable **2024.** The headset **2001** further includes one or more irradiation sources **2008** arranged on the positioning structure **2002.** Also, the headset **2001** includes a second fastening structure **2034** configured to fasten the headset **2001** to a head of a user, when in use.

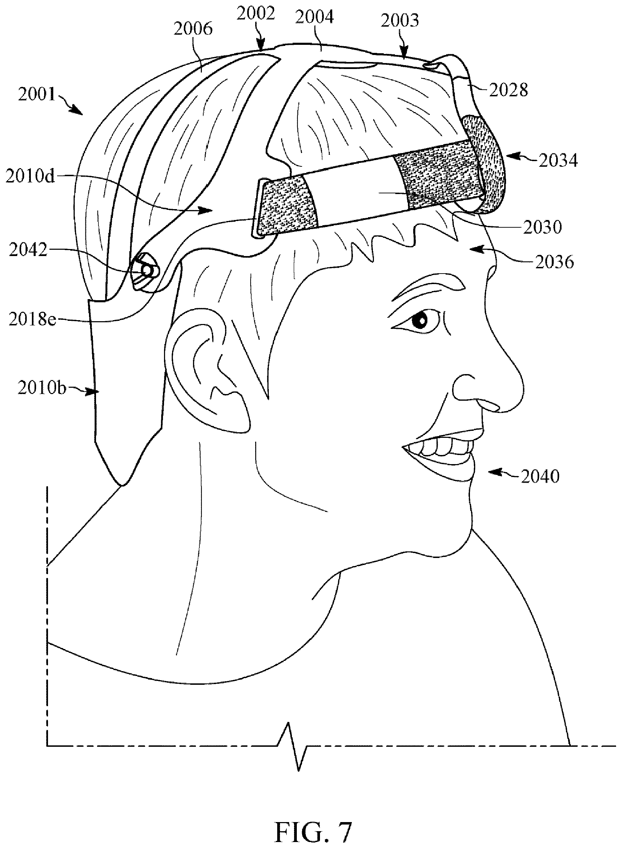

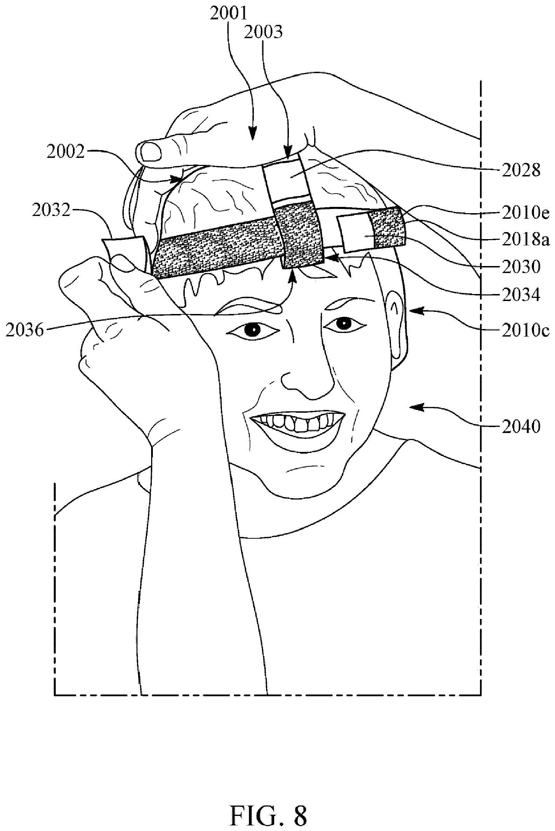

**Fig. 6** illustrates a front perspective view of the headset **2001** of **FIG. 3** in use. **FIG. 7** illustrates a right-side perspective view of the headset **2001** of **FIG. 3** in use. **FIG. 8** illustrates a front perspective view of the second fastening structure **2034** of the headset **2001** of **FIG. 3****.** The second fastening structure **2034** is in the form of a T strap with ends including loop and hook fasteners. A head portion **2028** of the second fastening structure **2034** connects with the notch **2003** of the central segment **2004** to then loop around a body portion **2030** of the second fastening structure **2034** to fasten the body portion **2030** upwardly across a periphery of the forehead **2036** of the user **2040.** Further, a transverse fastening strap **2032** fastens the body portion **2030** laterally around the periphery of the forehead **2036.** The body portion **2030** of the second fastening structure **2034** aligns and therefore connects with the aperture **2018e** of an expanded portion **2010d** and with the aperture **2018a** of an expanded portion **2010e**.

The second fastening structure **2034** advantageously allows the user **2040** to tighten the headset **2001** around the head of the user **2040** and tighten and secure the central segment **2004** over the head of the user **2040.** In other variations not illustrated herein, the second fastening structure **2034** may be in the form of, for example, hook-and-loop fasteners, a buckle fastening structure and/or other types of adjustable fastening/strapping mechanisms applicable in the art. In several alternate embodiments, the second fastening structure **2034** may include a combination of joining mechanisms or strapping mechanisms in the form of straps, nuts and bolts, or any alternative joining mechanisms or strapping mechanisms can be utilised to form the positioning structure **2002** around a portion of the head of the user **2040.**

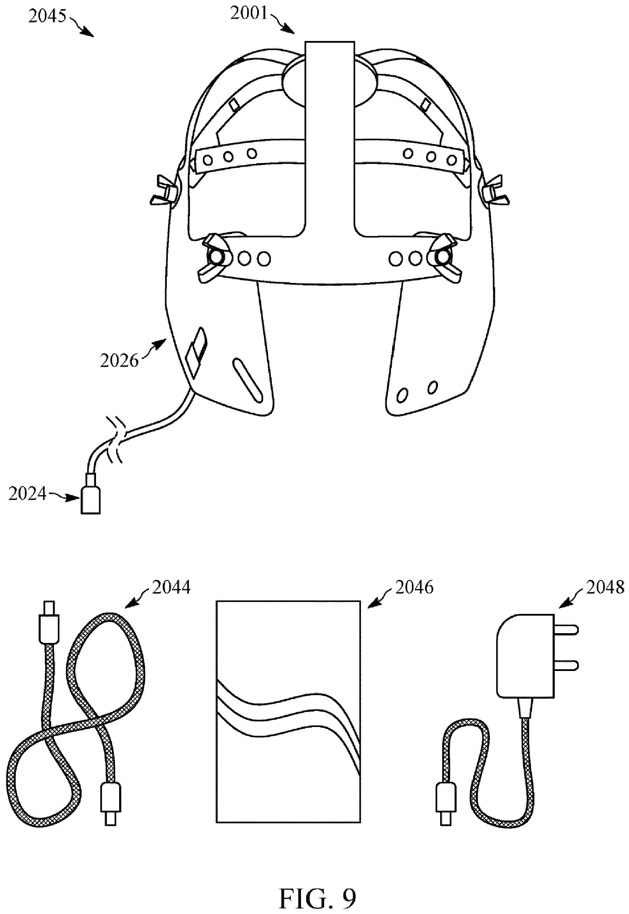

**FIG. 9** illustrates a perspective view of several components of a transcranial device **2045,** in accordance with an embodiment of the present invention. The headset **2001** may be connected via an inlet port **2026** to a power cable **2024.** The power cable **2024** may be connected with a male-to-male cable **2044** to a power source **2046** which may be charged or powered through a power cord **2048** with a power receptacle that would be plugged into an external power socket (not illustrated herein). In other words, the power source **2046** includes a power adaptor comprising an Analog to Digital (AC/DC) converter. The power adaptor is configured to receive power from an Alternating Current (AC) based power supply. As disclosed previously, in other variations the transcranial device **2045** can be powered by a portable power source such as an 8000 mAh Lithium Polymer.

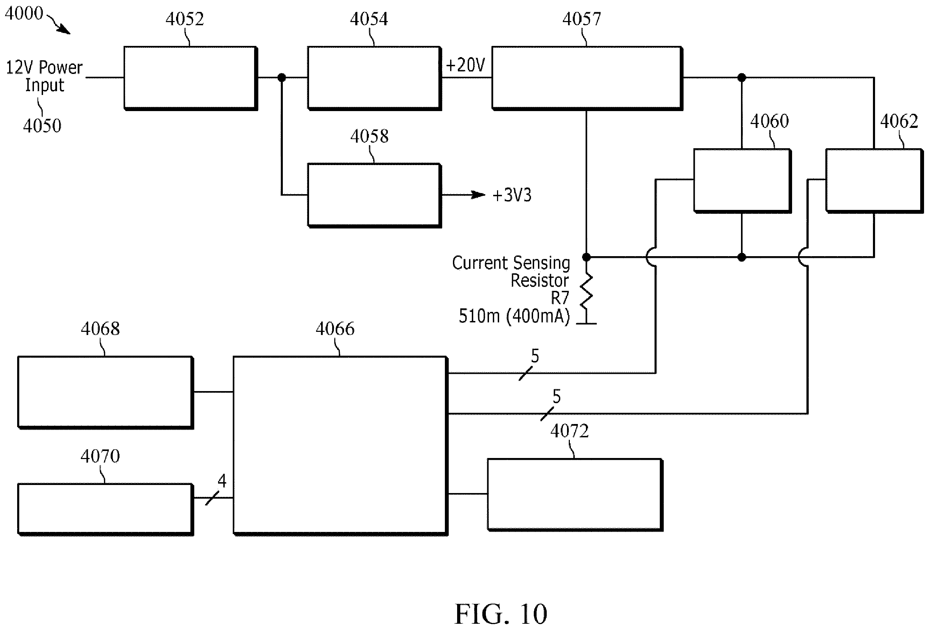

**FIG. 10** illustrates a logical block diagram of an electronic circuitry **4000** forming the control module **1022** of the transcranial device **2045,** in accordance with an embodiment of the invention. The electronic circuitry **4000** of the transcranial device **2045** incorporated into the transcranial device **2045** may consist of a processor **4066** (for example, in the form of a System on a Chip (SoC)) incorporating a Bluetooth Low Energy (BLE) module as a communication interface. The BLE module as the communication interface advantageously enables a wireless connection to other external programmable devices (such as a smartphone, tablet or computer device). Also, the processor **4066** may further include DC/DC power converters and one or more switching metal-oxide semiconductor field-effect transistors (MOSFET) to ensure that each LED is driven at the correct peak current, pulse frequencies and duty cycles coded in the form of machine-readable instructions stored in a memory unit and/or a storage device. The electronic components of the electronic circuitry **4000** consist of a temperature monitor **4068** and an audio transducer **4072.** The electronic components of the electronic circuitry **4000** can also have other modules embedded for various uses not disclosed herein such as, for example, Wi-Fi or WLAN modules, broadband cellular modules (enabling connections to, for example, 4G LTE or 5G), GPS modules and/or microphone sensors.

Further, a 12 V power source **4050** may power a supply conditioner **4052** which then powers a Boost regulator **4054** and 3.3V regulator **4058.** Power from the boost regulator **4054** is directed into an LED current regulator **4057** which then powers the array of infrared and red diode strings (or IR and RED LEDs - **4060** and **4062** respectively) acting as the one or more irradiation sources **1008** and/or **2008.** The IR and RED LEDS **(4060** and **4062** respectively) are controlled by the processor **4066.** The processor **4066** may also connect to the temperature monitor **4068,** a 4 -way Dual In-line Package (DIP) switch **4070** and the audio transducer **4072.** The 4-way DIP switch allows a user to select an operational state of the transcranial device **1000** and/or **2045** from up to sixteen (16) available preconfigured operational states.

In several alternate embodiments, not illustrated herein, the one or more irradiation sources **1008** and/or **2008** may be, for example, discretely or individually placed in separate locations. In other variations, the one or more irradiation sources **1008** and/or **2008** may be arranged to form, for example, clusters, columns, rows or arrays. The one or more irradiation sources **1008** and/or **2008** of the transcranial device **1000** and/or **2045** may be designed to pulse at 40 Hertz, a frequency similar to neural gamma waves. Advantageously, the neural gamma waves are correlated with increased memory activity, perception, cognition and creativity. The operating parameters of the irradiation sources **1008** are detailed in further detail later in this invention. In several alternate embodiments of the present invention, other types of the one or more irradiation sources **1008** and/or **2008** within the RGB spectrum, emitting pulses corresponding to other brain wave wavelengths, such as delta, theta, alpha or beta brain waves may be used. Each one of the one or more irradiation sources **1008** and/or **2008** may be embedded in a dome made from a suitable bio-compatible material (such as glass) which may advantageously make direct contact with the scalp of the user. Furthermore, the electronic circuitry **4000** is located in the central segment **1000** and is fully insulated from any contact with the scalp via a round protective pad lined with a non-conducting polymer material.

The substrate structure **1005** and/or **2005** of the positioning structure **1002** and/or **2002** of the transcranial device **1000** and/or **2045** may be made from a material with suitable biocompatible and advantageous thermal/mechanical properties (e.g. fibreglass), and may also be coated with a suitable impermeable solder resistant material (e.g. KSMS6189). Furthermore, the positioning structure **1002** and/or **2002** (in combination with the second fastening structure **2034** illustrated in **FIGS 6-8****)** may advantageously act as a heat sink whilst providing a higher degree of comfort and reducing the number of adjustments required to fit the headset **1001** and/or **2001** snugly to the head of the user.

In several embodiments, the transcranial device **1000** and/or **2045** may also come with a driver for a nasal application that is pulsed at 10 Hz which is a frequency similar to neural alpha waves. Alpha waves are correlated with the resting state of the brain, offering support for mental coordination, mindfulness and learning. **Table 1, Table 2**, **Table 3**, and **Table 4** below provide an example of one product-level implementation of the transcranial device **1000** and/or **2045** as disclosed in the application.

| **TABLE 1: EXAMPLE DESCRIPTION OF THE KEY FUNCTIONAL ELEMENTS** | |
|---|---|
| Wavelength | RED 630-670nm |
| | IR 810nm |
| Timing | 12 minutes RED and then 12 minutes IR |
| Pulse Rate | 40 Hz (Period 25 millisecond) |
| | Duty Cycle of 16.67% |
| | Peak drive current 400 mA |
| Safety | Temp sensor. Turns OFF or modulates sensor supplies if temp sensor > 50°C. Max Device Temperature is 31 °C |

| **TABLE 2: STIMULUS SITES** | |
|---|---|
| 20 Locations overall, each has: | x1 IR (810 nm) LEDs |
| | x1 Red (630 nm - 670 nm) LEDs |

| **TABLE 3: OPERATING CHARACTERISTICS** | |
|---|---|
| Multiple Segments, 5 in all, each containing x4 Red and x4 IR LEDs | |
| Segment Timing: | 40 Hz pulse rate (25 msec period) |
| | Each segment energized for 5.0 msec (20% duty cycle) |
| | Peak Drive Current 400 mA |

| **TABLE 4: AVERAGE OUTPUT POWER PER LED** | |
|---|---|
| RED LED (630-670nm) | Peak current = 385 mA |
| | Peak Electrical Power = 780 mW |
| | Peak Pulse Optical Power = 135 mW |
| | Average Optical Power = 27 mW |
| | Energy per pulse = 0.675 mJ |
| IR LED (810nm) | Peak current = 385 mA |
| | Peak Electrical Power = 1,117 mW |
| | Peak Pulse Optical Power = 260 mW |
| | Average Optical Power = 52 mW |
| | Energy per pulse = 1.3 mJ |

Points of stimulation for the transcranial device **1000** and/or **2045** may be selected points correlating with points that have previously been found to modulate the default mode network. The remaining posterior and sub-occipital points of the cranium, mastoid process, C1/C2 relate to the occipital and C2 sympathetic nerve that are also convergent on the trigeminal/cervico nuclei in the brain stem and well as being physically superficial points to the cervical and suboccipital lymphatic system systems of the vessels. These points were designed to change cortical connectivity and light-activated switching of ion channels, receptor kinases and other light-sensitive opsins that could produce signalling in downstream molecular pathways. The brain's glymphatic and lymphatic fluid flow are also under circadian control as well as being a very important layer in control of neurophysiology of the CNS and in Parkinson's disease. This may be augmented by physical treatment and of note, these are multimodal sensory receptors that also respond to light.

Parkinson's Disease can be considered a disease of mitochondrial insufficiency and altered brain, connectome activity, and disruption of normal brain oscillation symptoms (Swann, de Hemptinne et al. 2016), especially gamma and beta oscillations, with a reduction and lack of ATP energy that is available for interneuron firing and fast-spiking of neurons (Yao, Wu et al. 2020). The consequential disruption of Basal Ganglia and motor networks that connect these areas of the brain, including the Substantia Nigra, Thalamus and Brainstem was assessed to be a prime target of the points chosen for the headset **1000** and/or **2045.** These are activated by light through absorption by neuropsin in areas where the light can penetrate (Suzuki, Kanagawa et al. 2014) and would involve the nodes of Ranvier (Arancibia-Carcamo and Attwell 2014).

The de-activation of the default mode network and the activation of the salience network and their connectivity to the Endorestiform Nucleus were considered to be the main target areas. The Brainstem, as well as the Cervico Trigeminal Nucleus, predicted the points that would be effective in the de-activation of the default mode network and the activation of the salience network and their connectivity to the Endorestiform Nucleus (Paxinos and Huang 2013, Li, Liao et al. 2021). The 20 points for locating the red and infrared radiation emitting irradiation sources **1008** and/or **2008** were selected to be points correlating with points that have previously been found to modulate the default mode network (Saltmarche, Naeser et al. 2017, El Khoury, Mitrofanis et al. 2019, Zomorrodi, Loheswaran et al. 2019).

Various modifications to these embodiments are apparent to those skilled in the art, from the description and the accompanying drawings. The principles associated with the various embodiments described herein may be applied to other embodiments. Therefore, the description is not intended to be limited to the embodiments shown along with the accompanying drawings but is to provide the broadest scope consistent with the principles and the novel and inventive features disclosed or suggested herein. Accordingly, the invention is anticipated to hold on to all other such alternatives, modifications, and variations that fall within the scope of the present invention and appended claims.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. A transcranial device for irradiating a cranium of a user, the transcranial device comprising:
a power source; and
a headset configured to receive power from the power source, the headset comprising:
a positioning structure comprising one or more irradiation sources and a control module located on a substrate structure, wherein the control module is configured to modify operational characteristics of the one or more irradiation sources, the substrate structure comprising:
a central segment, and
one or more ribs extending longitudinally and outwardly from the central segment, the one or more ribs include one or more respective proximal ends attached to the central segment and one or more respective distal ends located longitudinally at one or more respective predetermined distances from the central segment, the one or more ribs comprising one or more respective expanded portions at the one or more respective distal ends of the one or more respective ribs,
wherein the one or more ribs are pliable, such that, the one or more ribs are configured to be bent for locating and fastening the headset onto a head of a user, using one or more first fastening structures and a second fastening structure.

2. The transcranial device as claimed in claim 1, wherein the power source comprises one or more rechargeable batteries.

3. The transcranial device as claimed in claim 1, wherein the power source comprises a power adaptor comprising an Analog to Digital (AC/DC) converter, the power adaptor configured to receive power from an Alternating Current (AC) based power supply.

4. The transcranial device as claimed in claim 1, wherein the power source is attached to the positioning structure.

5. The transcranial device as claimed in claim 1, wherein the one or more irradiation sources is selected from a group consisting of Light Emitting Diodes (LEDs), lasers, and incandescent sources of electromagnetic radiation.

6. The transcranial device as claimed in claim 1, wherein the plurality of irradiation sources is configured to emit electromagnetic radiation of red-light wavelengths (630 nm - 670 nm) and Infrared radiation wavelengths (800 nm - 820 nm).

7. The transcranial device as claimed in claim 6, wherein the one or more irradiation sources comprise twenty (20) LEDs configured to emit red light of wavelengths ranging between 630 nm and 670 nm, and twenty (20) LEDs configured to emit infrared radiation of wavelengths ranging between 800 nm and 820 nm.

8. The transcranial device as claimed in claim 1, wherein the control module is located in the central segment.

9. The transcranial device as claimed in claim 1, wherein the control module is covered with a protective pad made from a non-conducting material.

10. The transcranial device as claimed in claim 9, wherein the non-conducting material is selected from a group consisting of Acrylonitrile Butadiene Styrene (ABS), Polyvinyl Chloride (PVC), High-Density Polyethylene (HDPE), Low-Density Polyethylene (LDPE), and combinations thereof.

11. The transcranial device as claimed in claim 1, wherein the control module comprises a communication interface configured to wirelessly connect to an external programmable device.

12. The transcranial device as claimed in claim 1, wherein the control module is configured to operate the one or more irradiation sources in pulsating mode with a pulsating frequency of 40 Hz and a peak drive current of 400 mA.

13. The transcranial device as claimed in claim 1, wherein the control module further comprises a four (4) -way Dual In-line Package (DIP) switch configured to allow the user to select an operational state from sixteen (16) preconfigured operational states.

14. The transcranial device as claimed in claim 1, wherein the substrate structure is made up of fibreglass and coated with an impermeable solder-resistant material.

15. The transcranial device as claimed in claim 1, wherein the one or more ribs comprise:
a first rib extending rearwardly from the central segment, the first rib configured to extend over a frontal bone and an occipital bone of the cranium,
a second rib extending rearwardly and obliquely towards a right side when in use, the second rib configured to extend over a right parietal bone, a right temporal bone, a right mastoid process and the occipital bone of the cranium,
a third rib extending rearwardly and obliquely towards a left side when in use, the third rib configured to extend over a left parietal bone, a left temporal bone, a left mastoid process and the occipital bone of the cranium,
a fourth rib extending laterally towards the right side when in use, the fourth rib configured to extend over the frontal bone and a right sphenoid bone of the cranium, and
a fifth rib extending laterally towards the left side when in use, the fifth rib configured to extend over the frontal bone and a left sphenoid bone of the cranium.

16. The transcranial device as claimed in claim 1, wherein each one of the one or more expanded portions comprises one or more apertures, such that, for locating and fastening the headset onto the head of the user, respective apertures of the one or more expanded portions are configured to be overlapped and fastened using the one or more first fastening structures.

17. The transcranial device as claimed in claim 16, wherein the one or more first fastening structures comprise screws and/or bolts.

18. The transcranial device as claimed in claim 16, wherein the central segment comprises a notch, and the second fastening structure comprises a T-strap with ends provided with loop and hook fasteners, the T-strap comprising:
a head portion, and
a body portion,
wherein the head portion is configured to connect with the notch to loop around the body portion, to fasten the body portion upwardly across a periphery of a forehead of the user, and
wherein a transverse fastening strap is configured to fasten the body portion laterally around the periphery of the forehead.

19. The transcranial device as claimed in claim 1, further comprising a nasal driver for nasal application, the nasal driver configured to operate the one or more irradiation sources with a pulsating frequency of 10 Hz.

20. A transcranial device for irradiating a cranium of a user, the transcranial device comprising:
a power source; and
a headset configured to receive power from the power source, the headset comprising:
a positioning structure comprising one or more irradiation sources and a control module located on a substrate structure, wherein the control module is configured to modify operational characteristics of the one or more irradiation sources, the substrate structure comprising:
a central segment, and
one or more ribs extending longitudinally and outwardly from the central segment, the one or more ribs include one or more respective proximal ends attached to the central segment and one or more respective distal ends located longitudinally at one or more respective predetermined distances from the central segment, the one or more ribs comprising one or more respective expanded portions at the one or more respective distal ends of the one or more respective ribs,
wherein the one or more ribs are pliable, such that, the one or more ribs are configured to be bent for locating and fastening the headset onto a head of a user, using one or more first fastening structures and a second fastening structure, and
wherein the one or more ribs comprise:
a first rib extending rearwardly from the central segment, the first rib configured to extend over a frontal bone and an occipital bone of the cranium,
a second rib extending rearwardly and obliquely towards a right side when in use, the second rib configured to extend over a right parietal bone, a right temporal bone, a right mastoid process and the occipital bone of the cranium,
a third rib extending rearwardly and obliquely towards a left side when in use, the third rib configured to extend over a left parietal bone, a left temporal bone, a left mastoid process and the occipital bone of the cranium,
a fourth rib extending laterally towards the right side when in use, the fourth rib configured to extend over the frontal bone and a right sphenoid bone of the cranium, and
a fifth rib extending laterally towards the left side when in use, the fifth rib configured to extend over the frontal bone and a left sphenoid bone of the cranium.
